(19) Europäisches Patentamt
European Patent Office
Office européen des brevets

(11) Veröffentlichungsnummer: **0 056 933**
**B1**

(12) # EUROPÄISCHE PATENTSCHRIFT

(45) Veröffentlichungstag der Patentschrift: **14.12.83**

(51) Int. Cl.³: **C 07 C 45/42,** C 07 C 49/255

(21) Anmeldenummer: **82100046.0**

(22) Anmeldetag: **07.01.82**

(54) **Verfahren zur Herstellung von 1-Aryloxy-methylketonen.**

(30) Priorität: **16.01.81 DE 3101143**

(43) Veröffentlichungstag der Anmeldung: **04.08.82 Patentblatt 82/31**

(45) Bekanntmachung des Hinweises auf die Patenterteilung: **14.12.83 Patentblatt 83/50**

(84) Benannte Vertragsstaaten: **AT BE CH DE FR GB IT LI NL**

(56) Entgegenhaltungen:
**BULLETIN DE LA SOCIETE CHIMIQUE DE FRANCE, Nr. 7, 1972, Seiten 2749-2755 C. RIVALLE et al.: No. 439. Transformation des cétones alpha-aryloxylées par la chaleur. III. Etude des alpha-aryloxypinacolones diversement substituées**
**TETRAHEDRON LETTERS, Nr. 22, 1978, Seiten 1943-1946 S.F. MARTIN et al.: "A facile procedure for the hydrolysis of vinyl halides to ketones"**

(73) Patentinhaber: **BAYER AG, Zentralbereich Patente, Marken und Lizenzen, D-5090 Leverkusen 1, Bayerwerk (DE)**

(72) Erfinder: **Jautelat, Manfred, Dr., Muellersbaum 28, D-5093 Burscheid 1 (DE)**
Erfinder: **Stetter, Jörg, Dr., Gellertweg 4, D-5600 Wuppertal 1 (DE)**
Erfinder: **Arlt, Dieter, Dr. Prof., Rybniker Strasse 2, D-5000 Köln 80 (DE)**

**EP 0 056 933 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung von 1-Aryloxy-methylketonen

Die vorliegende Erfindung betrifft ein neues Verfahren zur Herstellung von teilweise bekannten 1-Aryloxymethylketonen, die als Zwischenprodukte zur Synthese von fungiziden Wirkstoffen verwendet werden können.

Es ist bekannt, Monohalogenmethylketone mit Phenolen in Gegenwart geeigneter Basen zu 1-Aryloxymethylketonen umzusetzen (vgl. hierzu z.B. die DE-AS 2 201 063 und DE-AS 2 401 715). Die dabei benötigten Monohalogenmethylketone können durch Halogenierung von Methylketonen hergestellt werden (vgl. z.B. Houben-Weyl, «Methoden der Organischen Chemie», Band 7/2 c, S. 2162, Georg Thieme Verlag, Stuttgart (1977)). Dieses Verfahren hat den Nachteil, dass die Halogenierung und die Umsetzung mit Phenolen in zwei getrennten Schritten ausgeführt werden.

Es wurde nun gefunden, dass man die teilweise bekannten 1-Aryloxy-methylketone der allgemeinen Formel

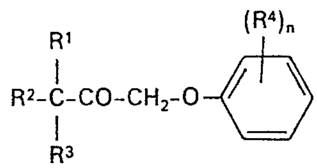

(I)

in welcher

$R^1$, $R^2$ und $R^3$ für Wasserstoff, für gegebenenfalls substituiertes Alkyl, für Alkenyl, Alkinyl und/oder für gegebenenfalls substituiertes Aryl stehen, oder auch $R^1$ und $R^2$ gemeinsam für eine Alkylen-Kette stehen können, und

$R^4$ für Halogen, Alkyl, Alkoxy, gegebenenfalls substituiertes Aryl und/oder Nitro steht und

n für die Zahlen 0, 1, 2 oder 3 steht,

in einem Eintopf-Verfahren erhält, indem man 1-Halogen-2-aryloxy-1-alkene der Formel

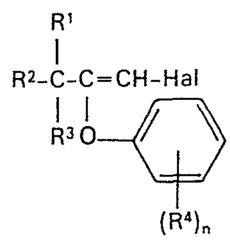

(II)

in welcher

Hal für Chlor oder Brom steht und $R^1$, $R^2$, $R^3$, $R^4$ und n die weiter oben genannte Bedeutung besitzen,

im Temperaturbereich zwischen +20 und 150°C einer sauren Hydrolyse unterwirft und anschliessend nach Hinzufügen basischer Stoffe bei schwach alkalischer Reaktion die Umsetzung im genannten Temperaturbereich zu Ende führt.

Die eingangs erwähnte bekannte Umsetzung von Monohalogenmethylketonen zu 1-Aryloxymethylketonen wird in wasserfreien Lösungsmitteln ausgeführt. Es ist deshalb als ausgesprochen überraschend zu bezeichnen, dass nach dem erfindungsgemässen Verfahren auch 1-Aryloxymethylketone in wasserhaltigem Reaktionsmedium erhalten werden können.

Das erfindungsgemässe Verfahren weist eine Reihe von Vorteilen auf. Die Durchführung der Reaktionsschritte in einem Eintopf-Verfahren bringt eine Vereinfachung des Verfahrens und ermöglicht dadurch eine Senkung der Herstellungskosten. Ferner sind die Ausgangsstoffe, 1-Chlor-2-aryloxy-1-alkene (II), aus preiswerten, technisch gut zugänglichen Rohstoffen wie Isobuten, 2-Methyl-2-buten und Isopren gut herstellbar. Nach dem erfindungsgemässen Verfahren können auch ungesättigte 1-Aryloxy-methylketone synthetisiert werden, die über die bekannte Chlorierung entsprechender Methylketone nicht zugänglich sind.

Verwendet man beispielsweise 1-Chlor-2-(4-chlorphenoxy)-3,3-dimethyl-1-buten als Ausgangsstoff, so kann der Reaktionsablauf durch das folgende Formelschema wiedergegeben werden:

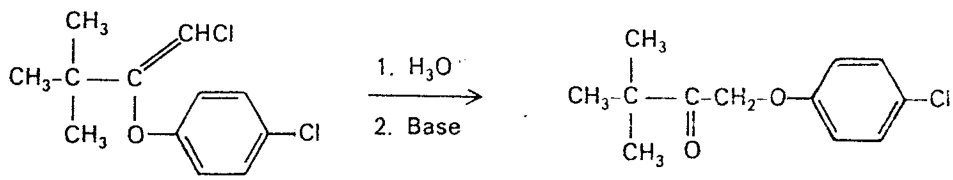


Die als Ausgangsstoffe zu verwendenden 1-Chlor-2-aryloxy-1-alkene sind durch die allgemeine Formel (II) definiert. In dieser Formel stehen $R^1$ und $R^2$, die gleich oder verschieden sein können, vorzugsweise für Alkyl, Alkenyl und Alkinyl mit jeweils bis zu 4 Kohlenstoffatomen und für Phenyl und Halogenphenyl. Die Alkyl- und die Phenyl-Gruppen können gegebenenfalls substituiert sein durch Halogen, Nitro, Phenyl, Halogenphenyl, Alkoxy mit bis zu 3 Kohlenstoffatomen und durch Phenoxy oder Halogenphenoxy. Weiterhin können $R^1$ und $R^2$ gemeinsam eine Alkylenkette mit vorzugsweise 2 bis 7 Kohlenstoffatomen bilden. $R^3$ steht vorzugsweise für Alkyl mit bis zu 10, insbesondere bis zu 6 Kohlenstoffatomen und für Aryl mit 6 bis 10 Kohlenstoffatomen, insbesondere für Phenyl. Die Alkyl- oder Aryl-Reste können gegebenenfalls die oben bei der Besprechung der $R^1$ und $R^2$-Reste vorzugsweise genannten Substituenten tragen. $R^4$ steht vorzugsweise für Chlor, Fluor, Alkyl oder Alkoxy mit jeweils bis zu 3 Kohlenstoffatomen und für Phenyl und Halogenphenyl. Hal steht vorzugsweise für Chlor, und n nimmt vorzugsweise die Zahlenwerte 0, 1 und 2 an.

Als Beispiele für die erfindungsgemäss zu verwendenden 1-Halogen-2-aryloxy-1-alkene der Formel (II) seine im einzelnen genannt:

1-Chlor-2-phenoxy-3,3-dimethyl-1-buten
1-Chlor-2-(4-chlorphenoxy)-3,3-dimethyl-1-buten
1-Chlor-2-(2,4-dichlorphenoxy)-3,3-dimethyl-1-buten
1-Chlor-2-(2,4,6-trichlorphenoxy)-3,3-dimethyl-1-buten
1-Chlor-2-(4-fluorphenoxy)-3,3-dimethyl-1-buten
1-Chlor-2-(2-chlorphenoxy)-3,3-dimethyl-1-buten
1-Chlor-2-(2-fluorphenoxy)-3,3-dimethyl-1-buten
1-Chlor-2-(2-chlor-4-fluorphenoxy)-3,3-dimethyl-1-buten
1-Chlor-2-(2-methyl-4-chlorphenoxy)-3,3-dimethyl-1-buten
1-Chlor-2-(4-(p-chlorphenyl)-phenoxy)-3,3-dimethyl-1-buten
1-Chlor-2-phenoxy-3,3-dimethyl-1-penten
1-Chlor-2-(4-chlorphenoxy)-3,3-dimethyl-1-penten
1-Chlor-2-(4-fluorphenoxy)-3,3-dimethyl-1-penten
1-Chlor-2-(2,4-dichlorphenoxy)-3,3-dimethyl-1-penten
1-Chlor-2-phenoxy-3,3-dimethyl-1,4-pentadien
1-Chlor-2-(4-chlorphenoxy)-3,3-dimethyl-1,4-pentadien
1-Chlor-2-(4-fluorphenoxy)-3,3-dimethyl-1,4-pentadien
1-Chlor-2-(2,4-dichlorphenoxy)-3,3-dimethyl-1,4-pentadien
1-Chlor-2-phenoxy-3,3-dimethyl-5-fluor-1-penten
1-Chlor-2-(4-chlorphenoxy)-3,3-dimethyl-5-fluor-1-penten
1-Chlor-2-(2,4-dichlorphenoxy)-3,3-dimethyl-5-fluor-1-penten
1-Chlor-2-phenoxy-3,3-diethyl-1-penten
1-Chlor-2-(4-chlorphenoxy)-3,3-diethyl-1-penten
1-Chlor-2-(4-fluorphenoxy)-3,3-diethyl-1-penten
1-Chlor-2-(2,4-dichlorphenoxy)-3,3-diethyl-1-penten
1-Chlor-2-phenoxy-3-methyl-1-buten
1-Chlor-2-(chlorphenoxy)-3-methyl-1-buten
1-Chlor-2-(4-fluorphenoxy)-3-methyl-1-buten
1-Chlor-2-(2,4-dichlorphenoxy)-3-methyl-1-buten
1-Chlor-2-(4-chlorphenoxy)-3,3,4-trimethyl-1-penten
1-Chlor-2-(4-chlorphenoxy)-3,3,5,5-tetramethyl-1-hexen
1-Chlor-2-(4-chlorphenoxy)-3,3-dimethyl-1-octen
1-Chlor-2-(4-chlorphenoxy)-3-methyl-3-(4-chlorphenyl)-1-buten
1-Chlor-2-(4-chlorphenoxy)-3-methyl-3-(2,4-dichlorphenyl)-1-buten
1-Chlor-2-(4-chlorphenoxy)-3,3-dimethyl-pent-1-en-4-in
1-Chlor-2,5-di-(4-chlorphenoxy)-3,3-dimethyl-1-penten
1-Chlor-2,5-di(2,4-dichlorphenoxy)-3,3-dimethyl-1-penten
1-(2-Chlor-1-[4-chlorphenoxy]-vinyl)-1-methylcyclohexan
1-(2-Chlor-1-[4-chlorphenoxy]-vinyl)-1-ethylcyclopentan
1-(2-Chlor-1-[4-chlorphenoxy]-vinyl)-1-methylcyclopropan

Die genannten 1-Halogen-2-aryloxy-1-alkene können in einfacher Weise aus Olefinen durch Halogenwasserstoff-Addition, nachfolgende Addition an Vinylidenhalogenid in Gegenwart von sauren Katalysatoren (z.B. Aluminiumchlorid) und Umsetzung des erhaltenen Adduktes mit Alkaliphenolaten erhalten werden.

Als Verdünnungsmittel kommen für das erfindungsgemässe Verfahren gegenüber den Reaktionspartnern inerte organische Lösungsmittel in Frage. Vorzugsweise werden polare Lösungsmittel wie Alkohole, so z.B. Methanol, Ethanol, Ethylenglykol, ferner Ether wie Tetrahydrofuran oder Dioxan, sodann Ketone, wie Aceton, Methylethylketon, ferner Acetonitril, Dimethylformamid, Dimethylsulfoxid und N-Methylpyrrolidon verwendet; weiterhin können Mischungen oder Lösungen bestehend aus organischen Lösungsmitteln (wie z.B. Alkoholen oder Ketonen) und Wasser Anwendung finden. Auch ist die Verwendung eines Zweiphasensystems (organisches Lösungsmittel/Wasser) mit üblichen Phasentransfer-Katalysatoren (z.B. Ammonium- oder Phosphoniumsalzen) möglich.

Die Hydrolyse wird mit Säuren wie Chlorwasserstoffsäure, Ameisensäure, Essigsäure, Trifluoressigsäure, Oxalsäure, Schwefelsäure, Methansulfonsäure oder p-Toluolsulfonsäure durchgeführt; die Säuren können in wässriger Verdünnung vorliegen. Bevorzugt wird Salzsäure verwendet. Die Hydrolyse erfolgt im Temperaturbereich zwischen +20 und 150°C, vorzugsweise zwischen 40 und 100°C.

Als zu erfindungsgemässen Umsetzung benötigte basische Stoffe kommen sowohl anorganische wie organische Basen in Betracht. Vorzugsweise werden Alkali- und Erdalkalicarbonate wie z.B. Natriumcarbonat, Kaliumcarbonat und Calciumcarbonat, Alkali- und Erdalkalihydroxide wie Natriumhydroxid, Kaliumhydroxid, Calciumhydroxid und Magnesiumhydroxid, Alkalihydrogencarbonate wie Natriumhydrogencarbonat, Erdalkalioxide wie Calciumoxid oder Amine wie Triethylamin verwendet.

Die Umsetzung wird nach Hinzufügen der genannten basischen Stoffe bei Temperaturen zwischen +20 und 150°C, vorzugsweise bei 40 bis 100°C, zu Ende geführt. Sie kann sowohl bei Normaldruck im offenen System als auch unter Druck im Autoklaven erfolgen.

Zur Durchführung der sauren Hydrolyse setzt man auf 1 Mol 1-Halogen-2-aryloxy-1-alken der Formel II vorzugsweise eine äquimolare Menge an einbasischer Säure ein. Nach der Hydrolyse wird durch Zugabe von vorzugsweise 2 Mol Alkalicarbonat, wie z.B. Natriumcarbonat oder Kaliumcarbonat, das Reaktionsgemisch schwach alkalisch gestellt. Die Aufarbeitung erfolgt durch Zugabe eines mit Wasser nicht mischbaren Lösungmittels und Waschen des Reaktionsgemisches mit Wasser, Trocknen der organischen Phase und Abziehen des Verdünnungsmittels. Das erhaltene Produkt der Formel (I) kann, wenn notwendig, durch Destillation gereinigt werden. Die Reaktion wird als Eintopfverfahren ausgeführt, kann jedoch auch in zwei getrennten Stufen erfolgen.

Die nach dem erfindungsgemässen Verfahren zugänglichen 1-Aryloxy-methylketone der Formel sind geeignete Zwischenprodukte zur Herstellung von fungizid wirksamen Azol-Derivaten. Sie können durch Halogenierung in 1-Halogen-1-aryloxy-methylketone überführt und anschliessend mit Imidazol oder 1,2,4-Triazol umgesetzt werden. Die Umsetzung mit z.B. Triazolen führt zu Verbindungen, die eine hohe Wirksamkeit gegen phytopathogene Pilze besitzen (vgl. hierzu die DE-AS 2201 063 oder die entsprechende US-PS 3912 752).

Die nachfolgenden Beispiele erläutern das erfindungsgemässe Verfahren.

**Herstellungsbeispiele:**

Beispiel 1

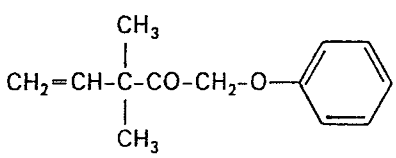

22,2 g (0,1 mol) 1-Chlor-2-phenoxy-3,3-dimethyl-1,4-pentadien werden im Gemisch aus 10ml Aceton und 10 ml konz. Salzsäure während 3 Stunden unter Rückfluss erhitzt. Dann setzt man 27,6 g (0,2 mol) Kaliumcarbonat zu, verdünnt mit weiteren 65 ml Aceton und erhitzt während 19 Stunden unter Rückfluss. Die Lösung wird mit Methylenchlorid verdünnt und mit Wasser mehrfach ausgeschüttelt. Nach dem Trocknen über Natriumsulfat zieht man das Lösungsmittel ab und erhält 20,1 g Rohprodukt. Die Destillation bei Kp. $_{0,5}$/80–83°C liefert 16,9 g 1-Phenoxy-3,3-dimethyl-pent-4-en-2-on. Die Ausbeute beträgt 83% der Theorie.

NMR ($CDCl_3$): $\alpha$ 1,3 (s, 6H), 4,8 (s, 2H), 5,0–6,2 (m, $-CH{=}CH_2$), 6,7–7,4 (m, 5H)

Vorprodukte

VP 1 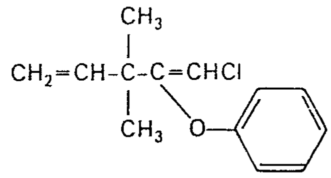

116 g (1 mol) Natriumphenolat und 82,5 g (0,5 mol) 1,1-Dichlor-3,3-dimethyl-1,4-pentadien werden in 500 ml Dimethylformamid während 8 Stunden unter Rückfluss erhitzt. Die Lösung wird mit Methylenchlorid verdünnt und mit verdünnter Natronlauge ausgeschüttelt. Nach Trocknen der organischen Phase über Natriumsulfat wird das Lösungsmittel im Vakuum abgezogen. Es bleiben 108,5 g Rohprodukt zurück, das destilliert wird. Bei Kp. $_{0,5}$/80–90°C gehen 93,6 g 1-Chlor-3,3-dimethyl-2-phenoxy-1,4-pentadien über. Die Reinausbeute beträgt 84% der Theorie.

NMR ($CDCl_3$): $\delta$1,25 (s, 6H), 4,9–6,2 ($-CH{=}CH_2$), 5,85 (s, 1H), 6,8 – 7,4 (m, 5 H)

Das benötigte 1,1-Dichlor-3,3-dimethyl-1,4-pentadien wird wie folgt erhalten. 1,3-Dichlor-3-methyl-butan wird im Temperaturbereich zwischen −10 und +5°C in Gegenwart von Aluminiumchlorid mit 1,1-Dichlorethen umgesetzt. Dabei erhält man 3,3-Dimethyl-1,1,5-trichlor-1-penten, das durch Destillation gereinigt wird. Durch Chlorwasserstoff-Abspaltung mit Chinolin im Temperaturbereich zwischen 200 und 240°C wird 1,1-Dichlor-3,3-dimethyl-1,4-pentadien erhalten.

Beispiel 2

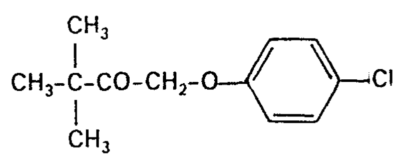

12,25 g (0,05 mol) 1-Chlor-3,3-dimethyl-2-(4′-chlorphenoxy)-1-buten werden in 10 ml Aceton und 5 ml konz. Salzsäure während 7 Stunden unter Rückfluss erhitzt. Dann gibt man 13,8 g (0,1 mol) Kaliumcarbonat und 30 ml Aceton hinzu und erhitzt während weiteren 7,5 Stunden unter Rückfluss. Die Suspension wird mit Methylenchlorid verdünnt und mit Wasser mehrfach ausgeschüttelt. Nach Trocknen über Natriumsulfat und Entfernen des Lösungsmittels erhält man 11,34 g Rohprodukt, das bei Kp. $_{0,15}$/108–128°C destilliert wird und anschliessend erstarrt. Man erhält 10,2 g 1-(4′-Chlorphenoxy)-3,3-dimethyl-butan-2-on vom Fp. 58–60°C, das sind 90% der Theorie.

NMR ($CDCl_3$): δ 1,2 (s, 9H), 4,85 (s, 2H) 5,7–7,3 (m, 4H).

Vorprodukt

VP 2 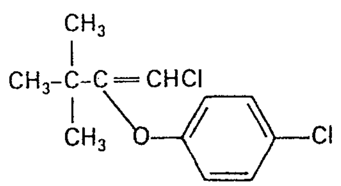

26 g (0,2 mol) p-Chlorphenol werden in 150 ml (Dimethylformamid mit 40 ml 30% Natriummethylat-Lösung (0,2 mol) umgesetzt. Nach Abziehen des Methanols werden bei 20 mbar noch 50 ml Dimethylformamid abdestilliert. Dann gibt man 15,3 g (0,1 mol) 1,1-Dichlor-3,3-dimethyl-1-buten hinzu und erhitzt während 18 Stunden unter Rückfluss. Die Lösung wird mit Methylenchlorid verdünnt und mit verdünnter Natronlauge dreimal ausgeschüttelt. Die organische Phase wird über Natriumsulfat getrocknet, und das Lösungsmittel im Vakuum entfernt. Die Destillation liefert im Vorlauf Dimethylformamid und als Hauptfraktion bei Kp. $_{0,15}$/110–128 °C 20 g 1-Chlor-3,3-dimethyl-2-(4′-chlorphenoxy)-1-buten, das sind 81% der Theorie.

NMR ($CDCl_3$): δ 1,15 (s, 9H), 5,9 (s, 1 H), 6,8 – 7,4 (m, 4H).

**Patentansprüche**

1. Verfahren zur Herstellung von 1-Aryloxy-methylketonen der allgemeinen Formel

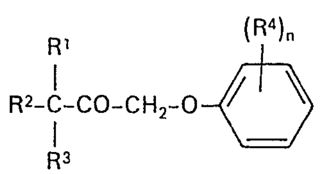 (I)

in welcher
$R^1$, $R^2$ oder $R^3$ für Wasserstoff, für gegebenenfalls substituiertes Alkyl, für Alkenyl, Alinyl und oder für gegebenenfalls substituiertes Aryl stehen, oder auch $R^1$ und $R^2$ gemeinsam für eine Alkylen-Kette stehen können, und
$R^4$ für Halogen, Alkyl, Alkoxy, gegebenenfalls substituiertes Aryl und/oder Nitro steht, und
n für die Zahlen 0, 1, 2 oder 3 steht,
in einem Eintopf-Verfahren, dadurch gekennzeichnet, dass man 1-Halogen-2-aryloxy-1-alkene der Formel

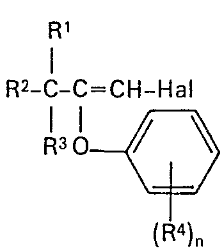 (II)

in welcher
Hal für Chlor oder Brom steht und
$R^1$, $R^2$, $R^3$, $R^4$ und n die weiter oben genannte Bedeutung besitzen,
im Temperaturbereich zwischen +20 und 150°C einer sauren Hydrolyse unterwirft und anschliessend nach Hinzufügen basischer Stoffe bei schwach alkalischer Reaktion die Umsetzung im genannten Temperaturbereich zu Ende führt.

2. Verfahren nach Anspruch 1, dadurch gekennzeichnet, dass man die saure Hydrolyse und die anschliessende Umsetzung jeweils im Temperaturbereich zwischen 40 und 100°C durchführt.

**Revendications**

1. Procédé pour la fabrication de 1-aryloxyméthylcétones de formule générale

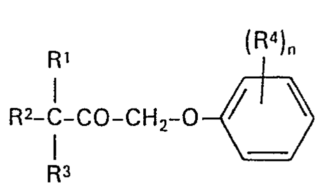 (I)

dans laquelle
$R^1$, $R^2$ ou $R^3$ représentent l'hydrogène, un alkyle éventuellement substitué, un alcényle ou alcynyle et/ou un aryle éventuellement substitué, ou bien $R^1$ et $R^2$ peuvent représenter ensemble une chaîne alkylène, et
$R^4$ représente un halogène ou un alkyle, alcoxy, un aryle éventuellement substitué et/ou un groupe nitro, et
n représente les nombres 0, 1, 2 ou 3,
dans un procédé en un pot, caractérisé en ce que l'on soumet des 1-halogéno-2-aryloxy-1-alcènes de formule

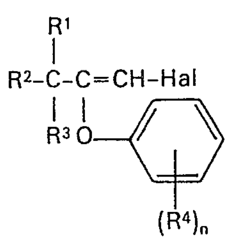 (II)

dans laquelle
Hal représente le chlore ou le brome, et
$R^1$, $R^2$, $R^3$, $R^4$ et n ont la signification indiquée ci-dessus, à une hydrolyse acide dans un domaine de températures entre +20 et 150°C et on termine la réaction dans le domaine de températures mentionné avec réaction faiblement alcaline après addition de substances basiques.

2. Procédé selon la revendication 1, caractérisé en ce que l'on effectue l'hydrolyse acide et la réaction ultérieure chaque fois dans un domaine de températures entre 40 et 100°C.

**Claims**

1. Process for the preparation of 1-aryloxymethyl ketones of the general formula

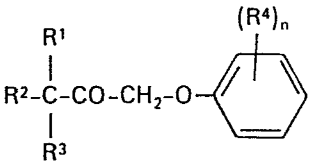

(I)

in which

$R^1$, $R^2$ or $R^3$ represent hydrogen, optionally substituted alkyl, or alkenyl, alkinyl and or optionally substituted aryl, or

$R^1$ and $R^2$ together can represent an alkylene chain, and

$R^4$ represents halogen, alkyl, alkoxy, optionally substituted aryl and/or nitro and

n represents the number 0, 1, 2 or 3, in a one-pot process, characterised in that 1-halogeno-2-aryloxy-1-alkenes of the formula

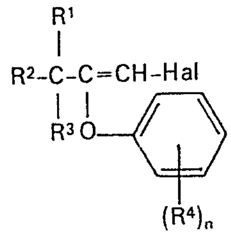

(II)

in which

Hal represents chlorine or bromine and

$R^1$, $R^2$, $R^3$, $R^4$ and n have the above mentioned meaning,

are subjected to acid hydrolysis in the temperature range between +20 and 150°C and, after basic substances have been added, the reaction is then brought to completion under weakly alkaline conditions in the temperature range mentioned.

2. Process according to Claim 1, characterised in that the acid hydrolysis and the subsequent reaction are each carried out in the temperature range between 40 and 100°C.